Europäisches Patentamt

European Patent Office

Office européen des brevets

(19)

(11) **EP 1 287 849 A1**

(12) ## DEMANDE DE BREVET EUROPEEN

(43) Date de publication:
**05.03.2003 Bulletin 2003/10**

(51) Int Cl.$^7$: **A61N 1/37**

(21) Numéro de dépôt: **02292119.1**

(22) Date de dépôt: **28.08.2002**

(84) Etats contractants désignés:
**AT BE BG CH CY CZ DE DK EE ES FI FR GB GR IE IT LI LU MC NL PT SE SK TR**
Etats d'extension désignés:
**AL LT LV MK RO SI**

(30) Priorité: **28.08.2001 FR 0111147**

(71) Demandeur: **ELA MEDICAL**
**92541 Montrouge (FR)**

(72) Inventeurs:
• **Casset, Cyril**
  **75010 Paris (FR)**
• **Limousin, Marcel**
  **75014 Paris (FR)**

(74) Mandataire: **Dupuis-Latour, Dominique**
**SEP Pagenberg & Associés**
**14 boulevard Malesherbes**
**75008 Paris (FR)**

(54) **Dispositif médical actif implantable, notamment stimulateur cardiaque, défibrillateur, cardioverteur ou dispositif multiste, comprenant des moyens d'ajustement cycle à cycle de l'amplitude de stimulation**

(57) Le dispositif comprend des moyens de stimulation ventriculaire, des moyens d'ajustement de l'amplitude de stimulation, des moyens pour évaluer périodiquement un seuil d'entraînement et définir une amplitude de sécurité (Vs), et des moyens pour déterminer la détection ou la perte de capture sur un cycle cardiaque après stimulation à une amplitude de stimulation (V) donnée. Il est en outre prévu des moyens pour définir une amplitude de capture (Vc) supérieure ou égale au seuil d'entraînement mais inférieure à l'amplitude de sécurité. Les moyens d'ajustement sont aptes à : réduire temporairement l'amplitude de stimulation au-dessous de l'amplitude de sécurité jusqu'à la valeur d'amplitude de capture (Vc) ; vérifier, immédiatement après la stimulation à cette amplitude réduite, la détection ou la perte de capture (étapes 12,14) ; en cas de détection de capture, établir pour le prochain cycle cardiaque l'amplitude de stimulation à la valeur de l'amplitude de capture (étape 16) ; et, en cas de perte de capture, définir une nouvelle valeur, supérieure à la valeur courante, de l'amplitude de capture (étapes 44, 46).

FIG_1

EP 1 287 849 A1

## Description

L'invention concerne les "dispositifs médicaux implantables actifs" tels que définis par la directive 90/385/CEE du 20 juin 1990 du Conseil des communautés européennes, plus précisément les dispositifs stimulateurs cardiaques, défibrillateurs et/ou cardioverteurs permettant de délivrer au coeur des impulsions électriques de faible énergie pour le traitement des troubles du rythme cardiaque.

Elle concerne plus particulièrement l'ajustement de l'amplitude (niveau de tension) des impulsions de stimulation au cours du temps.

Le niveau de stimulation des cavités cardiaques (ventricule ou oreillette) est une valeur typiquement comprise entre 1,5 et 7,5 V, ajustable par pas de 0,5 V. Cette amplitude doit bien entendu être suffisamment élevée pour provoquer la dépolarisation du myocarde ; il faut cependant éviter des valeurs trop élevées pour ménager la durée de vie de la pile, car l'énergie de stimulation appliquée, et donc la consommation correspondante du dispositif, est proportionnelle au carré de l'amplitude (et également à la durée) de l'impulsion.

Le test du seuil d'efficacité de la stimulation ou "seuil d'entraînement" peut être effectué à intervalles réguliers, par exemple toutes les six heures, par mise en oeuvre d'un algorithme de test automatique décrit notamment dans le WO-A-93/02741 (Ela Médical). L'amplitude des impulsions de stimulation est ensuite ajustée sur la base du seuil ainsi mesuré, avec une marge de sécurité importante : le niveau ajusté est généralement le double de la valeur du seuil, ce niveau étant borné par un minimum (typiquement 2,5 V) et par un maximum (typiquement 5 V). Ce niveau sera appelé par la suite "amplitude de sécurité" et désigné Vs.

L'un des buts de l'invention est de réduire encore la tension de stimulation, jusqu'à un niveau proche de la tension de seuil (tension en dessous de laquelle il n'y aura plus de capture), en vérifiant bien entendu de façon beaucoup plus fréquente, typiquement à chaque cycle cardiaque, si la stimulation a été efficace ou non, de manière à réajuster cette tension de stimulation réduite et/ou rebasculer sur une tension correspondant à l'amplitude de sécurité. Cette tension réduite, voisine du seuil d'entraînement, sera appelée par la suite "amplitude de capture" et désignée Vc.

Une telle technique est particulièrement avantageuse, car elle permet de s'affranchir de la marge de sécurité importante sur l'amplitude de stimulation, et donc d'allonger de façon substantielle la durée de vie de la pile.

En revanche, dans la mesure où la stimulation se fait à un niveau proche de celui du seuil d'entraînement, il est indispensable d'opérer un test de capture "cycle à cycle", c'est-à-dire d'examiner à chaque cycle cardiaque - et non plus à intervalles périodiques, par exemple toutes les six heures - si la stimulation a été efficace ou non.

Si une perte de capture est détectée, une contre-stimulation à énergie plus importante doit être appliquée immédiatement (c'est-à-dire au bout des 63 ms suivant la stimulation inefficace) afin de compenser sans attendre l'absence de dépolarisation du myocarde ; de plus, la prochaine stimulation sera opérée sur la base de l'amplitude de sécurité, et le niveau d'amplitude de capture sera réévalué pour déterminer s'il y a lieu de le réajuster à la hausse.

Il y a cependant lieu de tenir compte du fait que la détection d'une perte de capture peut en réalité n'être que la conséquence de la survenue d'une "fusion", c'est-à-dire d'une stimulation intervenant de façon concomitante à une dépolarisation ventriculaire spontanée.

En effet, après une stimulation auriculaire, l'événement ventriculaire détecté (complexe QRS) peut être soit le résultat direct de cette stimulation compte tenu du temps de latence existant entre ces deux événements, soit un complexe spontané survenant dans la même fenêtre temporelle (fusion).

La survenue d'une fusion peut avoir un effet délétère du point de vue hémodynamique, du fait de la présence de deux excitations myocardiques très rapprochées dont l'une est inutile. Dans le cas d'un test de capture, même si une fusion n'a pas d'effet hémodynamique, elle est néanmoins susceptible de produire artificiellement une surélévation de la valeur du seuil d'entraînement mesuré par rapport au seuil réel du patient, avec pour conséquence un réajustement de l'amplitude de stimulation à un niveau excessif, maintenu au moins pendant plusieurs heures ; bien qu'il ne soit pas en lui-même dangereux, un tel niveau excessif constitue une source de surconsommation et donc de réduction de la durée de vie de l'implant.

Il y a donc lieu, lors de la détection d'une perte de capture, de discriminer entre : une véritable perte de capture consécutive à une augmentation naturelle du seuil d'entraînement, la survenue d'une fusion avérée, ou encore un simple cycle atypique (détection ventriculaire post-auriculaire, cycle trop rapide ou extrasystole), ces deux derniers cas ne justifiant pas une révision immédiate à la hausse de l'amplitude de stimulation.

La présente invention propose à cet effet un mode de détermination de l'amplitude de capture en fonction du seuil d'entraînement détecté, tenant compte des possibles survenues de fusions ou de cycles atypiques, et assurant si nécessaire un basculement automatique vers l'amplitude de sécurité.

Plus précisément, le dispositif de l'invention est d'un type connu comprenant : des moyens de stimulation ventriculaire, pour délivrer au coeur des impulsions électriques présentant une amplitude et une durée prédéterminées ; des moyens d'ajustement de l'amplitude de ces impulsions de stimulation ; des moyens pour évaluer à intervalles périodiques un seuil d'entraînement et définir une amplitude de sécurité correspondante ; et des moyens de détection de capture cycle à cycle, pour déterminer la détection ou la perte de capture sur un cycle cardiaque après une stimulation à une amplitude de stimulation donnée.

De façon caractéristique de l'invention, il est en outre prévu des moyens pour définir une amplitude de capture à un niveau fonction du seuil d'entraînement, supérieur ou égal à ce seuil d'entraînement mais inférieur à l'amplitude de sécurité, et les moyens d'ajustement de l'amplitude des impulsions de stimulation sont des moyens aptes à : réduire temporairement l'amplitude de stimulation au-dessous de l'amplitude de sécurité jusqu'à ladite valeur d'amplitude de capture ; vérifier, immédiatement après la stimulation à cette amplitude réduite, la détection ou la perte de capture ; en cas de détection de capture, établir pour le prochain cycle cardiaque l'amplitude de stimulation à la valeur de l'amplitude de capture ; et, en cas de perte de capture, définir une nouvelle valeur, supérieure à la valeur courante, de l'amplitude de capture.

Les moyens pour définir l'amplitude de capture peuvent notamment établir cette amplitude de capture à un niveau égal au seuil d'entraînement augmenté d'un incrément fixe, par exemple un pas d'ajustement de l'amplitude de stimulation.

Très avantageusement, les moyens d'ajustement sont des moyens aptes, en cas de perte de capture et avant d'établir l'amplitude de stimulation à la valeur de l'amplitude de sécurité, à opérer une discrimination entre, d'une part, la survenue d'une fusion ou d'un cycle atypique et, d'autre part, une élévation du seuil de capture, notamment par réduction de l'intervalle d'échappement ou du délai atrio-ventriculaire, puis par détection de capture sur une stimulation consécutive opérée à une amplitude correspondant à l'amplitude de capture.

De préférence, les moyens d'ajustement sont en outre des moyens aptes, en cas de survenue d'une fusion ou d'un cycle atypique, à opérer une discrimination additionnelle entre, d'une part, la survenue d'une fusion et, d'autre part, la survenue d'un cycle atypique, notamment par allongement de l'intervalle d'échappement ou du délai atrio-ventriculaire, puis par détection de capture sur une stimulation consécutive opérée à une amplitude correspondant à l'amplitude de sécurité. En cas de fusion, l'amplitude de stimulation est de préférence établie à la valeur de l'amplitude de sécurité et, en cas de cycle atypique, le rétablissement de la capture est détecté sur un nombre prédéterminé de cycles successifs ultérieurs et, dans un tel cas, l'amplitude de stimulation est établie à la valeur de l'amplitude de capture pour les prochains cycles cardiaques.

On va maintenant décrire un exemple de mise en oeuvre de l'invention, en référence à la figure unique annexée qui est un organigramme des différentes étapes de l'algorithme mis en oeuvre par le dispositif de l'invention.

Essentiellement, l'invention propose, à la différence des techniques où l'amplitude de stimulation est réajustée à intervalles réguliers, d'opérer un test de capture "cycle à cycle" c'est-à-dire d'examiner à chaque cycle cardiaque si la stimulation a été efficace ou non et réajuster en conséquence, si nécessaire, l'amplitude de stimulation.

Plus précisément, cette technique est mise en oeuvre par les étapes successives consistant à :

- contrôler périodiquement le seuil de capture ventriculaire,
- choisir une amplitude de stimulation ventriculaire en fonction du résultat de ce test,
- vérifier sur chaque cycle stimulé si la capture est correcte,
- modifier l'amplitude de stimulation si la capture n'est pas confirmée, et
- tenter de faire apparaître la conduction spontanée si celle-ci est présente.

Ces différentes étapes sont mises en oeuvre en trois phases successives, dont les deux premières interviennent de la même manière que dans le cas d'un test de capture à intervalles réguliers :

a) calibration préalable, pour évaluer et supprimer l'effet de la polarisation de la sonde à l'interface coeur/électrode en déterminant une valeur de référence,

b) mesure du seuil d'entraînement par rapport à la valeur de référence préalablement obtenue, et

c) contrôle de la capture sur chaque cycle où un ventricule est stimulé, et réajustement éventuel de l'amplitude de stimulation.

Les étapes (a) et (b) de calibration et de mesure du seuil d'entraînement sont identiques à celles opérées dans les dispositifs connus, par exemple de la manière décrite dans le WO-A-93/02741 précité, l'invention ne modifiant pas la manière dont chacune de ces deux étapes est en elle-même mise en oeuvre.

En résumé, pour l'étape (a) de calibration, le stimulateur mesure le potentiel évoqué (ou, plus précisément, la moyenne de plusieurs valeurs de potentiel évoqué) pour des impulsions d'amplitudes différentes, par exemple deux impulsions à 2 V et 4 V. Un algorithme détermine la droite de régression entre ces deux valeurs et l'ordonnée à l'origine, ou intercept, de cette droite. Le seuil d'efficacité de la capture est fixé à une valeur fonction de cet intercept, par exemple 75 % de la valeur de l'intercept (car l'intercept surestime en fait la valeur réelle de la polarisation), et le seuil ainsi calculé constituera la valeur de référence pour la mesure du seuil de capture.

L'étape suivante (b) de test du seuil consiste à déterminer le franchissement du seuil de capture de manière à ajuster le niveau de l'amplitude de l'impulsion de stimulation par rapport au seuil d'entraînement, c'est-à-dire au niveau minimal permettant la capture. Cet ajustement est obtenu par réduction contrôlée progressive du niveau de l'amplitude sur plusieurs cycles successifs, détection de la disparition de la capture, puis établissement de l'amplitude à un niveau légèrement supérieur au seuil correspondant à cette disparition. Si l'algorithme s'est déroulé normalement (absence de cycles atypiques, d'extrasystoles auriculaires, de rythme trop rapide), il détermine une valeur correspondant au dernier seuil de capture efficace trouvé, appelé "seuil d'entraînement".

Par ailleurs, l'algorithme détermine la valeur de l'amplitude de sécurité Vs, par exemple égale au double de la valeur du seuil, cette valeur étant bornée par un minimum (typiquement 2,5 V) et par un maximum (typiquement 5 V).

La phase suivante (c), caractéristique de l'invention, consiste, après avoir ainsi déterminé une amplitude de sécurité Vs, à calculer une seconde amplitude, plus faible, que l'on appellera "amplitude de capture" Vc.

Cette amplitude de capture sera, dans le présent exemple, définie comme étant égale au niveau du seuil d'entraînement augmenté de 0,5 V, avec une valeur minimale de 1 V, soit :

$$Vc \text{ (en volts)} = Sup \text{ (1 ; seuil d'entraînement + 0,5).}$$

L'algorithme va ensuite piloter le stimulateur de manière à appliquer une impulsion de stimulation avec cette amplitude réduite Vc :

- si une capture consécutive est détectée, alors Vc constituera l'amplitude de stimulation,
- si, au contraire, ce niveau réduit provoque une perte de capture, l'amplitude de sécurité Vs sera utilisée en secours et le comportement du myocarde sera analysé pour déterminer s'il y a lieu ou non de relever le niveau initialement évalué de l'amplitude de capture Vc.

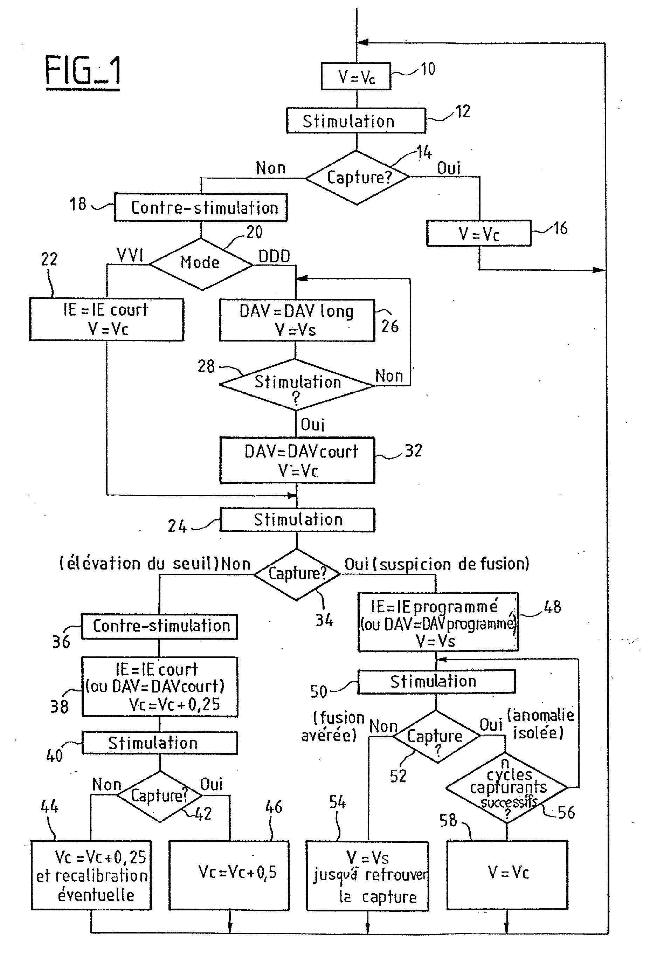

Le détail de l'algorithme est illustré sur la figure 1.

Tout d'abord (étape 10), l'amplitude de stimulation V est fixée au niveau de l'amplitude de capture Vc définie comme indiqué plus haut, à savoir Vc = Sup (1 ; seuil d'entraînement + 0,5). Bien entendu, si la valeur de l'amplitude de capture Vc a été corrigée suite à une précédente itération de l'algorithme, c'est cette valeur corrigée qui sera ici utilisée.

Le dispositif applique alors une impulsion de stimulation avec l'amplitude de capture ainsi définie (étape 12) et teste la présence ou l'absence d'une capture (étape 14) ; la détection d'une capture est effectuée, de manière en elle-même connue, par mesure de l'onde R dans la fenêtre de 63 ms suivant la délivrance de l'impulsion.

Si une capture est diagnostiquée, l'algorithme garde Vc comme amplitude de stimulation (étape 16).

Si, au contraire, une perte de capture est diagnostiquée, avant toute chose une contre-stimulation à énergie plus importante doit être appliquée immédiatement - c'est-à-dire au bout des 63 ms suivant la stimulation inefficace - afin de compenser sans attendre l'absence de dépolarisation du myocarde (étape 18).

L'étape suivante consiste à modifier certains paramètres de stimulation de manière à laisser s'exprimer, s'il est présent, le rythme ventriculaire propre du patient pour permettre de distinguer entre, d'une part, une véritable perte de capture due à un accroissement du seuil effectif d'entraînement, et, d'autre part, une perte de capture due à une autre cause, par exemple la survenue d'une fusion ou un cycle atypique.

Cette modification peut être opérée de deux manières, selon le mode de fonctionnement du stimulateur (testé en 20) :

- dans le cas d'un stimulateur fonctionnant en mode VVI ou équivalent (VVI, VVT, DDI) la modification porte sur la durée de l'intervalle d'échappement (IE) ventriculaire, qui est dans ce cas réduit, par exemple réduit d'une valeur de 63 ms, l'amplitude de stimulation étant toujours maintenue à la valeur d'amplitude de capture Vc (étape 22). Le myocarde est stimulé (étape 24) et le dispositif diagnostique alors la capture ou la perte de capture (étape 34).

- pour un stimulateur double-chambre opérant en mode DDD ou équivalent (DDD, DDTV, DD-CAM, VDD, etc.), agissant donc à la fois sur l'oreillette et le ventricule, le paramètre modifié est le délai atrio-ventriculaire (DAV), qui est forcé à une valeur longue, pour laisser au rythme ventriculaire spontané, s'il est présent, le temps de s'exprimer naturellement, avec une amplitude V = Vs assurant la capture (étape 26). Si, avec ces nouveaux paramètres, une stimulation est intervenue (étape 28), l'amplitude de stimulation est restaurée à la valeur d'amplitude de capture Vc et le DAV est forcé à une valeur courte, typiquement de 63 ms (étape 32) ; le myocarde est ensuite stimulé comme précédemment (étape 24) et le dispositif diagnostique alors la capture ou la perte de capture (étape 34). Si, à l'étape 28, aucune stimulation n'intervient, alors l'algorithme retourne à l'étape 26.

Si aucune capture n'est diagnostiquée à l'étape 34, ceci signifie qu'il y a eu augmentation du seuil, et que l'amplitude de capture Vc doit être réajustée.

Dans ce cas, avant toute chose une contre-stimulation est appliquée (étape 36).

Ensuite, tout en gardant les valeurs modifiées des paramètres IE ou DAV (IE court ou DAV court, comme aux étapes 22 ou 32), l'amplitude de capture Vc est augmentée d'un pas, typiquement un pas de 0,25 V (étape 38).

Une nouvelle stimulation est opérée sur la base de ces derniers paramètres (étape 40) et un test de capture est effectué (étape 42).

Si la perte de capture subsiste, l'amplitude de capture Vc est encore une fois augmentée et une phase de calibration peut être relancée de manière à réinitialiser la totalité des paramètres de l'algorithme d'ajustement de l'amplitude de stimulation. Toutefois, on fait en sorte que la calibration ne soit pas relancée plus de trois fois en six heures, et pas moins d'une heure avant la précédente calibration (étape 44).

Si une capture a été diagnostiquée à l'étape 42 après l'augmentation de l'amplitude de capture Vc à l'étape 38, alors l'algorithme établit comme amplitude de capture Vc, pour l'avenir, la nouvelle valeur déterminée à l'étape 38, c'est-à-dire la dernière amplitude minimale qui a permis d'obtenir une capture, avec une marge de sécurité de 0,5 V (étape 46).

Si, à l'étape 34, une capture a été diagnostiquée après modification de l'intervalle d'échappement à l'étape 22 ou du délai atrio-ventriculaire à l'étape 32, ceci signifie que le seuil n'a vraisemblablement pas augmenté, mais que la perte de capture à l'étape 14 était due à un phénomène tel qu'une fusion ou un cycle atypique.

Pour discriminer ces deux possibilités, l'algorithme restaure l'intervalle d'échappement ou le délai atrio-ventriculaire à la valeur programmée et choisit comme amplitude de stimulation non plus l'amplitude de capture Vc, mais l'amplitude de sécurité Vs telle que définie lors de la phase de calibration (étape 48). Une stimulation est alors appliquée sur ces nouvelles bases (étape 50) et la présence ou l'absence d'une capture consécutive est diagnostiquée (étape 52).

En cas de perte de capture, ceci signifie que l'on se trouve véritablement en situation de fusion, et le niveau de stimulation est maintenu à la valeur de l'amplitude de sécurité Vs jusqu'à ce qu'une capture puisse être diagnostiquée (étape 54). L'amplitude de sécurité est choisie pendant quelques cycles, pour éviter de perturber l'algorithme au cas où l'on se trouverait dans le cas d'une situation de fusion intermittente.

Si, au contraire, une capture a été diagnostiquée à l'étape 52, l'algorithme se poursuit en vérifiant que l'on a bien $n$ (typiquement, $n$ = 3) cycles successifs capturants, donc sans fusion (étape 56) et, si tel est effectivement le cas, le niveau de stimulation est rétabli à la valeur antérieure de l'amplitude de capture Vc (étape 58).

De façon générale, la description ci-dessus présuppose que le test de capture des étapes 14, 34, 42 et 52 fournit toujours un résultat non ambigu. Il existe cependant des situations dans lesquelles il n'est pas possible de vérifier la capture ou d'obtenir un diagnostic certain de la présence ou de la perte de la capture, par exemple dans les cas de détection ventriculaire post-auriculaire, ou en présence de cycles trop rapides, ou d'extrasystoles ventriculaires.

Dans ce cas, la stimulation suivante sera opérée avec l'amplitude de capture Vc comme niveau de stimulation, car c'est cette valeur Vc qui a été chargée dans le registre correspondant du dispositif ; mais pour augmenter l'énergie de stimulation, la largeur de l'impulsion sera augmentée, par exemple à 0,98 ms au lieu de 0,49 ms. Au cycle suivant, le dispositif chargera de toute façon, par précaution, l'amplitude de sécurité Vs pour la prochaine stimulation. Après quoi, le niveau de stimulation sera réduit à Vc, et ainsi de suite jusqu'à ce que l'algorithme puisse diagnostiquer de façon non ambiguë la capture ou la perte de capture.

## Revendications

1. Un dispositif médical actif implantable, notamment un stimulateur cardiaque, défibrillateur, cardioverteur ou dispositif multisite, comprenant :

   - des moyens de stimulation ventriculaire, pour délivrer au coeur des impulsions électriques présentant une amplitude et une durée prédéterminées,
   - des moyens d'ajustement de l'amplitude de ces impulsions de stimulation,
   - des moyens pour évaluer à intervalles périodiques un seuil d'entraînement et définir une amplitude de sécurité (Vs) correspondante, et
   - des moyens de détection de capture cycle à cycle, pour déterminer la détection ou la perte de capture sur un cycle cardiaque après une stimulation à une amplitude de stimulation (V) donnée,

   **caractérisé en ce que** :

   - il est en outre prévu des moyens pour définir une amplitude de capture (Vc) à un niveau fonction du seuil d'entraînement, supérieur ou égal à ce seuil d'entraînement mais inférieur à l'amplitude de sécurité (Vs),
   - les moyens d'ajustement de l'amplitude des impulsions de stimulation sont des moyens aptes à :

     - réduire temporairement l'amplitude de stimulation au-dessous de l'amplitude de sécurité jusqu'à ladite valeur d'amplitude de capture (Vc),
     - vérifier, immédiatement après la stimulation à cette amplitude réduite, la détection ou la perte de capture (étapes 12,14),
     - en cas de détection de capture, établir pour le prochain cycle cardiaque l'amplitude de stimulation à la valeur de l'amplitude de capture (étape 16), et
     - en cas de perte de capture, définir une nouvelle valeur, supérieure à la valeur courante, de l'amplitude de capture (étapes 44, 46).

**2.** Le dispositif de la revendication 1, dans lequel les moyens pour définir l'amplitude de capture (Vc) établissent cette amplitude de capture à un niveau égal au seuil d'entraînement augmenté d'un incrément fixe.

**3.** Le dispositif de la revendication 2, dans lequel, l'amplitude de stimulation étant ajustée par pas fixes, ledit incrément fixe est un incrément d'un pas.

**4.** Le dispositif de la revendication 1, dans lequel les moyens d'ajustement sont des moyens aptes, en cas de perte de capture et avant d'établir l'amplitude de stimulation à la valeur de l'amplitude de sécurité, à opérer une discrimination entre, d'une part, la survenue d'une fusion ou d'un cycle atypique et, d'autre part, une élévation du seuil de capture (étapes 22, 24, 34 ; 26, 28, 32, 24, 34).

**5.** Le dispositif de la revendication 4, où les moyens d'ajustement opèrent ladite discrimination par réduction de l'intervalle d'échappement ou du délai atrio-ventriculaire (étape 22, 32), puis par détection de capture sur une stimulation consécutive opérée à une amplitude correspondant à l'amplitude de capture (étapes 24, 34).

**6.** Le dispositif de la revendication 4, dans lequel les moyens d'ajustement sont des moyens aptes, en cas de survenue d'une fusion ou d'un cycle atypique, à opérer une discrimination additionnelle entre, d'une part, la survenue d'une fusion et, d'autre part, la survenue d'un cycle atypique (étapes 48, 50, 52).

**7.** Le dispositif de la revendication 6, où les moyens d'ajustement opèrent ladite discrimination additionnelle par allongement de l'intervalle d'échappement ou du délai atrio-ventriculaire (étape 48), puis par détection de capture sur une stimulation consécutive opérée à une amplitude correspondant à l'amplitude de sécurité (étapes 50, 52).

**8.** Le dispositif de la revendication 6, dans lequel les moyens d'ajustement sont des moyens aptes, en cas de fusion, à établir l'amplitude de stimulation à la valeur de l'amplitude de sécurité (étape 54).

**9.** Le dispositif de la revendication 6, dans lequel les moyens d'ajustement sont des moyens aptes, en cas de cycle atypique, à détecter le rétablissement de la capture sur un nombre prédéterminé de cycles successifs ultérieurs et, dans un tel cas, à établir pour les prochains cycles cardiaques l'amplitude de stimulation à la valeur de l'amplitude de capture (étapes 56, 58).

# FIG_1

- $V = V_c$ — 10
- Stimulation — 12
- Capture? — 14
  - Non
  - Oui
- Contre-stimulation — 18
- $V = V_c$ — 16
- Mode — 20
  - VVI
  - DDD
- IE = IE court, $V = V_c$ — 22
- DAV = DAV long, $V = V_s$ — 26
- Stimulation ? — 28
  - Non
  - Oui
- DAV = DAV court, $V = V_c$ — 32
- Stimulation — 24
- Capture? — 34
  - (élévation du seuil) Non
  - Oui (suspicion de fusion)
- Contre-stimulation — 36
- IE = IE programmé (ou DAV = DAV programmé), $V = V_s$ — 48
- IE = IE court (ou DAV = DAV court), $V_c = V_c + 0,25$ — 38
- Stimulation — 50
- Stimulation — 40
- Capture ? — 52
  - (fusion avérée) Non
  - Oui (anomalie isolée)
- Capture? — 42
  - Non
  - Oui
- n cycles capturants successifs ? — 56
- $V_c = V_c + 0,25$ et recalibration éventuelle — 44
- $V_c = V_c + 0,5$ — 46
- $V = V_s$ jusqu'à retrouver la capture — 54
- $V = V_c$ — 58

7

**Office européen des brevets**

# RAPPORT DE RECHERCHE EUROPEENNE

Numéro de la demande

EP 02 29 2119

## DOCUMENTS CONSIDERES COMME PERTINENTS

| Catégorie | Citation du document avec indication, en cas de besoin, des parties pertinentes | Revendication concernée | CLASSEMENT DE LA DEMANDE (Int.Cl.7) |
|---|---|---|---|
| X | US 4 895 152 A (CALLAGHAN FRANK J ET AL) 23 janvier 1990 (1990-01-23) * le document en entier * | 1-3 | A61N1/37 |
| Y | | 4-9 | |
| X | US 5 350 410 A (KLEKS JONATHAN A ET AL) 27 septembre 1994 (1994-09-27) * le document en entier * | 1-3 | |
| Y,D | US 5 411 533 A (DUBREUIL ANNE ET AL) 2 mai 1995 (1995-05-02) * le document en entier * | 4-9 | |

|  |
|---|
| **DOMAINES TECHNIQUES RECHERCHES (Int.Cl.7)** |
| A61N |

Le présent rapport a été établi pour toutes les revendications

| Lieu de la recherche | Date d'achèvement de la recherche | Examinateur |
|---|---|---|
| LA HAYE | 5 décembre 2002 | Ferrigno, A |

CATEGORIE DES DOCUMENTS CITES

X : particulièrement pertinent à lui seul
Y : particulièrement pertinent en combinaison avec un autre document de la même catégorie
A : arrière-plan technologique
O : divulgation non-écrite
P : document intercalaire

T : théorie ou principe à la base de l'invention
E : document de brevet antérieur, mais publié à la date de dépôt ou après cette date
D : cité dans la demande
L : cité pour d'autres raisons

& : membre de la même famille, document correspondant

EPO FORM 1503 03.82 (P04C02)

**ANNEXE AU RAPPORT DE RECHERCHE EUROPEENNE
RELATIF A LA DEMANDE DE BREVET EUROPEEN NO.**                  EP 02 29 2119

La présente annexe indique les membres de la famille de brevets relatifs aux documents brevets cités dans le rapport de recherche européenne visé ci–dessus.
Lesdits members sont contenus au fichier informatique de l'Office européen des brevets à la date du
Les renseignements fournis sont donnés à titre indicatif et n'engagent pas la responsabilité de l'Office européen des brevets.

05–12–2002

| Document brevet cité au rapport de recherche | | Date de publication | Membre(s) de la famille de brevet(s) | | Date de publication |
|---|---|---|---|---|---|
| US 4895152 | A | 23–01–1990 | DE | 3688070 D1 | 22–04–1993 |
| | | | DE | 3688070 T2 | 24–06–1993 |
| | | | EP | 0236562 A1 | 16–09–1987 |
| | | | US | 4858610 A | 22–08–1989 |
| US 5350410 | A | 27–09–1994 | AU | 5731694 A | 22–06–1994 |
| | | | EP | 0639993 A1 | 01–03–1995 |
| | | | JP | 7503175 T | 06–04–1995 |
| | | | WO | 9412237 A1 | 09–06–1994 |
| | | | US | 5417718 A | 23–05–1995 |
| US 5411533 | A | 02–05–1995 | FR | 2680093 A1 | 12–02–1993 |
| | | | EP | 0552357 A1 | 28–07–1993 |
| | | | WO | 9302741 A1 | 18–02–1993 |

EPO FORM P0460